# EUROPEAN PATENT APPLICATION

(11) **EP 2 570 427 A1**
(43) Date of publication of application: **20.03.2013**
(21) Application number: 12196741.8
(22) Date of filing: 14.01.2009
(51) Int. Cl.: C07K 14/44, A61K 39/008

(54) **Vaccine comprising a Ribosomal Protein Extract (RPE) and optionally a Th1-promoting adjuvant**

(30) Priority: 18.01.2008 US 21928 P; 18.01.2008 EP 08100634
(62) Divisional of application: 09701571.3
(71) Applicant: Laboratorios LETI, S.L. Unipersonal, 08038 Barcelona (ES)
(72) Inventor: Álvarez, Manuel Soto, ES-28916 Madrid (ES); Abánades, Daniel Ruiz, ES-28006 Madrid (ES); Bedate, Carlos Alonso, ES-28015 Madrid (ES)
(74) Representative: Hutter, Jacobus Johannes

(57) **Abstract**

The invention relates to composition comprising a RPE and optionally a Th₁-promoting adjuvant for the preparation of a medicament for the treatment or prevention of a parasitic disease and to its use.

SEQUENCE LISTING

<110>  Laboratorios LETI, S.L. unipersonal

<120>  Vaccine comprising a Ribosomal Protein Extract and a Th1
       promoting adjuvant

<130>  P6019394PCT

<150>  EP 08100634.8
<151>  2008-01-18

<150>  US 61/021,928
<151>  2008-01-18

<160>  2

<170>  PatentIn version 3.3

<210>  1
<211>  10
<212>  DNA
<213>  artificial

<220>
<223>  oligodeoxynucleotide

<400>  1
tcaacgttga                                                              10


<210>  2
<211>  14
<212>  DNA
<213>  artificial

<220>
<223>  oligodeoxynucleotide

<400>  2
gctagcgtta gcgt                                                         14

## Description

### Field of the invention

The invention relates to a composition comprising a ribosomal protein extract (RPE) and optionally a Th₁-promoting adjuvant for the preparation of a medicament for the treatment or prevention of a parasitic disease and to its use.

### Background of the invention

Leishmaniasis comprise several diseases caused by intracellular protozoan parasites belonging to the genus *Leishmania* that mainly infect macrophages of a variety of mammals including human and dogs. Depending largely on the species of the parasite and the immunocompetence state of the human host, the disease spectrum ranges from self-healing cutaneous leishmaniasis (CL) to fatal visceral leishmaniasis (VL) or kala-azar (18). Canine viscerocutaneous leishmaniasis (VCL) caused by *Leishmania infantum* and *L. chagasi* is an important emerging zoonosis found in countries around the Mediterranean basin, in the Middle East and in Latin America (16) being dogs the major reservoir of these parasites playing central role in the transmission to humans by phebotomine sand flies (47). Outcome of infection is determined by interactions between the host immune system and the different parasite species, yet the pathogenesis of leishmaniasis remains unclear and the knowledge on the mechanisms involved in the immune response to *Leishmania* in humans and dogs is still limited. Generally, protective immunity is associated with a classical cell mediated immune response that induce macrophage activation by T cells derived cytokines, while non-healing disease is associated with the generation of strong humoral responses (15, 26). Research for the development of second generation vaccines based on crude parasite fractions or based on defined parasite antigens was addressed to the identification of different surface or secreted parasite molecules that have been tested as vaccine candidates in several experimental models using diverse adjuvants (1, 17, 22, 46, 48, 49, 52, 54). The screening of expression libraries with sera from infected animals or humans has also enabled the selection of a few antigens as candidate vaccines (reviewed in (9)). Among them, those that elicit primarily a Th₁-type immune response in infected mice or human patient cells, irrespective of their cellular location, have been implicated in the generation of protective responses in different animal models (51, 55, 56). On the other hand, some of the isolated antigens are intracellular conserved proteins that predominantly stimulate humoral responses in human or dogs suffering VL or Th₂ mediated humoral responses in experimentally infected mice (3, 36, 38, 40, 42). The inadequate humoral response induced against them in dogs suffering leishmaniasis is thought to result in immunopathology, mainly due to the advert effects of immune complexes, particularly uveitis (13), lesions in the central nervous system (14) or nephritis (23, 24, 33, 34). Also, it has been recently shown that, the presence of IgG immune complexes in humans with VL correlates to an inability to resolve infections demonstrating that immune complexes can be detrimental to the infected host (30).

In spite of not being considered at first as good vaccine candidates, proteins that induce high humoral responses during the infectious process have been associated with the induction of protective responses. For example, parasite tubulins and the histone H2B were recognized by T-cell clones derived from a immune donor (39) and rK39 cause proliferation and IFN-γ production by T cells from immune mice (25). It has been also shown that genetic immunization with parasite H2B, H3 and H4 genes induces protection in murine visceral leishmaniasis models (27). Also, immunization of the receptor for activated C kinase (LACK) (32), some parasite cystein proteinases (38, 41) or the parasite nucleosome forming histones (11, 20) administered with Th₁-promoting adjuvants generates immune responses that correlates to protection against cutaneous leishmaniasis in murine models.

Among the evolutionary conserved antigens of *Leishmania,* several lines of evidence suggest that ribosomal proteins are immunologically relevant molecules during *Leishmania* infection. In some cases, ribosomal constituents can contribute to the host immune system dysfunction through their capacity to modulate cell activities and cytokine release during infection. Thus, injection of the *L. major* ribosomal protein S3a into BALB/c mice induced the polyclonal expansion of B-cell clones and inhibited T-cell proliferation (10). Also, genetic immunization with a DNA vaccine coding for the putative 60S ribosomal protein L31 exacerbate disease in mice models by the induction of IL-10 and Th₂ cytokines (44, 53). In addition, some parasite ribosomal proteins like the parasite acidic P proteins have been related with the generation of strong humoral responses in dogs and humans suffering leishmaniasis (reviewed in (42)).

Despite attempts so far, there is still no valuable vaccines against a parasitic disease such as Leishmaniasis. Therefore, there is still a need for such a vaccine.

### Description of the invention

In this work, we show that a RPE, especially a *Leishmania* RPE (LRPE) is a target of the immune response in dogs naturally infected with *L. infantum* and in mice experimentally infected with *L. major.* We further demonstrate that a strong Th₁ protective immune response is induced when a LRPE is coadministered with a Th₁-promoting adjuvant such as CpG oligodeoxynucleotide (ODN). Such compositions (a LRPE combined with a Th₁-promoting adjuvant) are very attractive to be used as a vaccine. The invention is further described below.

### Use

In a first aspect of the invention, there is provided the use of a RPE and optionally a Th₁-promoting adjuvant for the preparation of a medicament for the treatment or prevention of a parasitic disease in a subject.

In a preferred embodiment, a RPE is obtainable by carrying out the following steps using a parasite cell causing a parasitic disease when present in a subject:
a. mixing a parasite cell with a lysis buffer,
b. centrifuging the obtained mixture to obtain a cytosolic extract,
c. preparing a RPE from the obtained cytosolic extract.

In step a, a parasite preferably means a protozoa. Preferred parasites are defined later herein. More preferably, a protozoa is in the promastigote stage. The skilled person will know the amount of parasite cells approximately needed in order to prepare a desired amount of RPE. Typically for preparing approximately 500 micrograms of RPE, one will use approximately 3.10⁹ parasite cells. A lysis buffer is a buffer, which will break down at least some of the parasite cells. A preferred lysis buffer comprises a non-ionic surfactant. Good results were obtained with Nonidet P 40 (NP40) as non-ionic surfactant. However, other non-ionic surfactant may be used. A preferred lysis buffer used is as follows (Buffer A) : 10mM Tris HCl, pH 8.0, 150 mM NaCl, 1.5 mM MgCl₂ and 0.5% NP40 (Roche) and preferably supplemented with protease inhibitors such as PMSF 1mM, Leupeptin 8 µg/ml, Aprotinin 4 µg/ml and Pentatin 8 µg/ml). A suitable amount of parasite cells (approximately 10⁹ cells/ ml buffer A) is typically gently mixed with this lysis buffer using an eppendorf pipet.

In step b, at least one step of centrifugation at 4°C is applied on the obtained mixture of step a. Usually a first centrifugation step is carried out at approximately 3,000g for approximately 2 minutes. The obtained supernatant is preferably again centrifuged at approximately 13,000g for approximately 15 minutes at 4°C once or twice.

In step c, the obtained supernatant is used for preparing a RPE as described in (45). Briefly, the obtained supernatant is submitted to high speed centrifugation at approximately 90,000 rpm for approximately 30 min at 4 °C in a Beckman TL100.3 rotor. The obtained pellet is a crude ribosomal pellet, which is resuspended in buffer B (20 mM Tris-HCl, pH 7.4, 500 mM AcNH₄, 100 mMMgCL₂, 5mM β-mercaptoethanol) and centrifuged through a discontinuous sucrose gradient (20/40%) in buffer A at approximately 90,000 rpm at 4 °C in a TL100.3 rotor. The obtained pellet comprises ribosomes. This pellet is preferably dissolved in PBS (Phosphate Buffer Saline), sonicated and stored at - 70 °C.

Ribosomal proteins are well conserved cytosolic proteins. Therefore, a RPE as defined herein, may be prepared from any eukaryotic organism, be it plant or animal, be it from mammals, reptiles, fish, insects, or any other chromosome bearing organism, such as protozoa. Preferably a RPE is obtained from an organism which is close to the disease, preferably parasitic disease causing organism in the evolutionary tree. Therefore, of particular interest as a source of RPE to be used in the treatment of a parasitic disease are protozoans like plasmodium and in particular members of the trypanosomatid family, more in particular different species of the trypanosomatical protozoan *Leishmania.* There are over 20 known species of *Leishmania,* including species of the subgenus *Leishmania,* comprising the complex *L. major,* including *L. major,* the complex *L. Donovani,* including *L. chagasi, L. donovani* and *L. infantum,* the complex *L. Mexicana,* including *L. amazonensis* and *L. mexicana,* as well as the subspecies *Viannia,* comprising the complex *L. braziliensis,* including *L. braziliensis* and *L*. *peruviana* and the complex *L. guyanensis,* including *L. guyanensis* and *L. panamensis. Plasmodium* species of particular interest are *Plasmodium falciparum* and *Plasmodium vivax.* In a preferred embodiment, a RPE is obtained from a *Leishmania* species, preferably *Leishmania major* and/or *Leishmania infantum.* In another preferred embodiment, a RPE is obtained from a *Plasmodium* species. The skilled person will understand that a RPE may also be prepared by mixing a RPE from several distinct organims as identified herein. The use of a RPE in a vaccine instead of the use of a given protein is quite attractive since a RPE contains a large number of distinct antigens. Each of these antigens could potentially induce an immune protective response in a treated subject. Moreover, there are subjects that respond to antigen A and not to B and vice versa. Therefore, a vaccine as defined herein is intended to be used for a broad population of subjects since it contains a large number of distinct antigens. In a preferred embodiment, a RPE comprises at least one ribosomal protein and/or at least one antigen of a ribosomal protein and/or at least one protein fragment of a ribosomal protein. In a more preferred embodiment, a RPE comprises at least two ribosomal proteins and/or at least two antigens of a ribosomal protein and/or at least two protein fragments of a ribosomal protein. A protein fragment as defined herein is preferably a fragment comprising at least 2, 3, 5, 7, 10, 15, 20, 25, 30 or more contiguous amino acids of a corresponding ribosomal protein. In an embodiment, a RPE as defined herein does not comprise or does not consist of the acidic ribosomal protein P0 of *Leishmania infantum* and/or the ribosomal antigen LbeF4A from *Leishmania braziliensis.*

A Th₁-promoting adjuvant (like an adjuvant comprising a CpG ODN motif) is defined in the literature (Liu N., et al., (2003), Nature Immunology, 687-693 ) as an adjuvant which is able to promote or trigger a Th₁ immune response against a given antigen when used together with this antigen (here RPE) as detected in supernatants of splenocytes of a treated subject when cultured with the antigen. As control, the promotion or triggering of a Th1 immune response is assessed in a splenocyte population of the same subject which does not have been treated with the antigen and the adjuvant, or with same population only treated with the antigen. Triggering or promoting a Th₁ immune response is preferably defined by the induction of IFNγ as detected by culturing splenocytes of a treated subject with the antigen and/or by inducing the production of antigen specifc IgG2a immunoglobulines. The assessment of the induction of this cytokine is preferably carried out by ELISA on splenocytes as described in the example. The assessment of the induction of IgG2a is preferably carried out by ELISA or Western Blot as described in the example. The induction of IFNγ and/or IgG2a upon stimulation of splenocytes with RPE and an adjuvant preferably means that the adjuvant is qualified as a Th1-promoting adjuvant. Alternatively or in combination with the first definition of triggering or promoting a Th₁ immune response given above, triggering or promoting a Th₁ immune response may further be defined by the absence (or the absence of an induction) of a Th₂ immune response. A Th₂ immune response is characterised by a detectable increase in IL-4, IL-10 induction and/or the production of detectable IgG1 immunoglobulines when compared with non-treated splenocytes. The assessment of the induction of IL-4 and/or IL-10 is preferably carried out by ELISA on splenocytes as described in the example. The assessment of the induction of an IgG1 is preferably carried out by ELISA or Western Blot as described in the example.

Alternatively or in combination with the two first definitions of triggering or promoting a Th₁ immune response given above, triggering or promoting a Th₁ immune response may further be defined by the generation of an increase in IFNγ / IL-10 ratio and/or IFNγ / IL-4 ratio and /or a decrease in IgG1/IgG2a ratio against a defined antigen, in that case a RPE. In a preferred embodiment, a change (increase or decrease as indicated above) in any of these ratio of more than 2 indicates that an adjuvant has Th1 properties. The assessment of the induction of each of the mentioned cytokines is preferably carried out by ELISA on splenocytes as described in the example. The assessment of the induction of an immunoglobuline IgG1 or IgG2a is preferably carried out by ELISA or Western Blot as described in the example.

In a preferred embodiment, a Th-1 promoting adjuvant is or comprises or consists of an oligodeoxynucleotide. More preferably, an oligodeoxynucleotide (ODN) comprises or consists of CpG in which the C is non-methylated (CpG ODN): 3'purine-CpG-5'pyrimidine. A preferred oligodeoxynucleotide is or comprises or consists of a phosphorothioate-modified ODN sequence. The use of oligodeoxynucleotides having such modification is advantageous since the oligodeoxynucleotides hence used are more stable than non modified oligonucleotides and will not easily be degraded once they are in the blood system. Preferred Th-1 promoting adjuvant consists of or comprises at least one CpG motif , at least two or at least three. Preferred sequences of the immunostimulatory ODN (5' to 3') were TCAACGTTGA and GCTAGCGTTAGCGT. The skilled person is not limited to the sequences explicitly described herein. He may design other sequences and subsequently test them for their Th-1 promoting property as defined earlier herein. This preferred identified adjuvant CpG ODN is highly attractive since it was demonstrated in the example that the co-inoculation of LRPE with this Th1-promoting adjuvant induces protection against a challenge with *L. major* parasites in both BALB/c and C57BL/6 mouse strains. In both models, protection correlates to a specific production of IFN-γ. In BALB/c, a restriction in the production of IL-4 and IL-10 was also detected.

One advantage of the present invention is that it allows for the preparation of a medicament for the treatment of a broader spectrum of parasitic diseases i.e. a medicament with cross-species specificity. In many parasitic diseases, a vaccine raised against a specific species, only works against that specific species. One example of a parasitic disease in which this is the case is Leishmaniasis. At the moment, the disease is controlled by drugs, but drug treatment does not prevent the spread of the disease and in many cases is not very effective. In a preferred embodiment, a parasitic disease is leishmaniasis or malaria. More preferably, a parasitic disease is caused by a *Leishmania* or by a *Plasmodium* species. In a further preferred embodiment, a parasitic disease is caused by a different species than the species from which a RPE is derived. In particular, Leishmaniasis caused by one species from the genus *Leishmania* may be treated by using a composition based on a RPE from another *Leishmania* species. In one embodiment, Leishmaniasis caused by *L. major* is successfully treated with a composition comprising a RPE from *L. infantum.* Alternatively, other parasitic diseases, such as malaria, may be successfully treated with a composition based on a RPE of another species, for instance based on a RPE of *L. infantum.*

In the context of the invention, a subject means a human or an animal. An animal which is encompassed within the scope of the invention includes a mammal, preferably a dog.

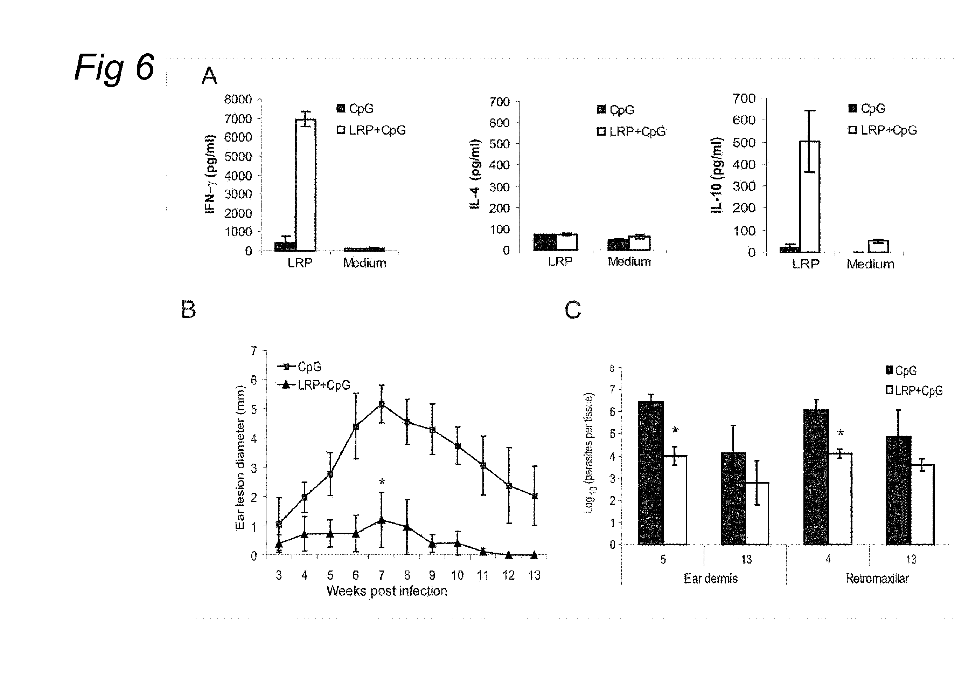

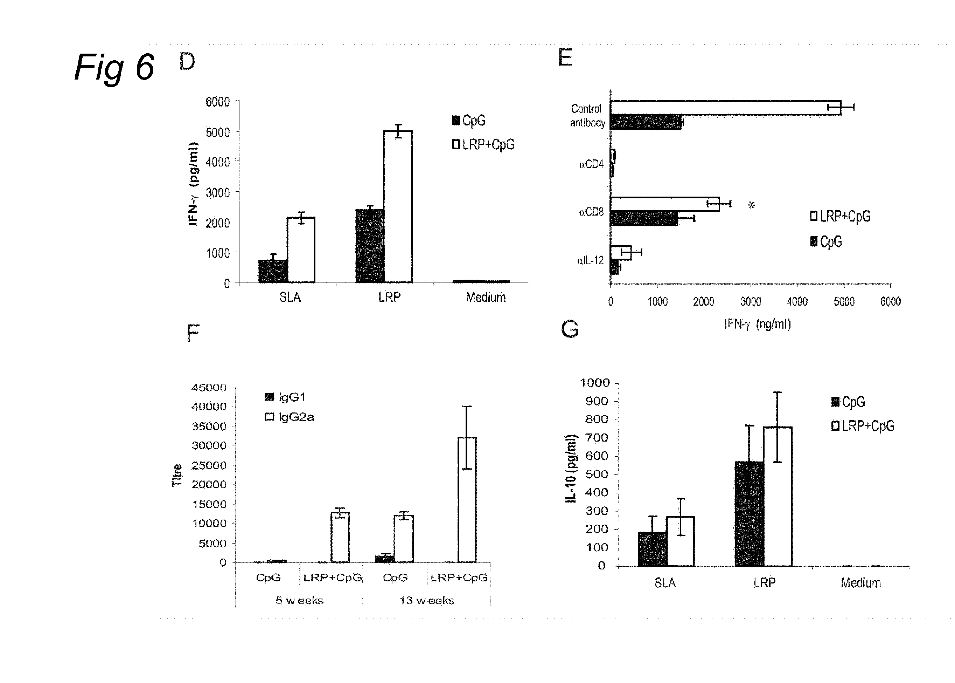

In a preferred embodiment, a medicament as defined herein is used to increase the ability of a human or animal immune system to fight against an infection and/or a disease, more preferably a parasitic infection and/or a parasitic disease. In particular, it may be used for administration to a human or animal subject. A medicament as defined herein is preferably administered parenterally, e.g. by injection or infusion by intravenous, subcutaneous, intraperitoneal, intramuscular, intraarterial or intralesional route. A preferred administration mode is subcutaneous. A medicament may be combined with a pharmaceutically acceptable medium or delivery vehicle by conventional techniques known in the art. For example, a RPE and optionally a Th₁-promoting adjuvant may be dissolved in Phosphate buffer saline (PBS). Methods for preparing parenterally administrable compositions are well known in the art and described in more details in various sources, including, for example, Remington's Pharmaceutical Sciences, Ed. AR Gennaro, 20th edition, 2000, Williams & Wilkins, PA, USA. A medicament is preferably administered in a therapeutically effective dose, i.e. one that will increase the ability of the human or animal immune system to fight an infection and/or a disease as defined herein. Preferably, a therapeutically effective dose of a medicament of the invention will prevent and/or delay the development of dermal lesion and/or induces a significant reduction of the parasite load in an ear and/or in a draining lymph node (DLN). The assessment of the presence of a dermal lesion is described in the legends of figure 6. The assessment of a parasite load is described in the example. A therapeutically effective dose of a medicament of the invention will preferably prevent the development of dermal lesion and/or will preferably induces a parasite load reduction in an ear of approximately 3 orders of magnitude and/or of approximately a similar magnitude in a DLN after a time period comprising first one vaccination using a composition of the invention followed by one sequential infection with a parasite and a waiting time of approximately ±6 weeks. In a preferred embodiment, a medicament as defined herein is a vaccine. In a more preferred embodiment, at least 12µg a RPE is being used in a vaccine. In an even more preferred embodiment, at least 12-20µg of a RPE must be used to provide an immune response optionally in combination with at least 50µg of a Th₁-promoting adjuvant such as for example, CpG ODN. A vaccine as defined herein may be a prophylactic or a therapeutic vaccine. The volume in which a RPE and optionally a Th1 promoting adjuvant may be dissolved may vary from 100-500 microliters.

### Composition

In a further aspect, there is provided a composition comprising a RPE and optionally a Th₁-promoting adjuvant. RPE and Th₁-promoting adjuvant have already been defined herein. In a preferred embodiment, a composition consists of a RPE and a Th₁-promoting adjuvant. A preferred Th₁-promoting adjuvant is a CpG ODN. A preferred composition comprises or consists of a RPE and optionally a Th₁-promoting adjuvant dissolved in PBS. In a further preferred embodiment, it is also encompassed by the present invention that a RPE and a Th1-promoting adjuvant are sequentially administered. Therefore, both components do not need to be physically present in one single composition as long as they are both administered to a subject.

Such composition may further comprise a pharmaceutically acceptable adjuvant and/or carrier.

Such composition is preferably for use as a medicament. The medicament is preferably a vaccine. Medicament and vaccine have already been extensively defined herein.

### Method

In another aspect, the invention provides for a method to prevent and/or treat a parasitic disease and/or delay its progression and/or prevent and/or delay the development of dermal lesion and/or induces a significant reduction of the parasite load in an ear and/or in a draining lymph node (DLN) all as defined herein. In this method, a vaccine of the invention functions as a therapeutic vaccine. Typically, there is a time period between infection and disease. In this case, a vaccine would act as a pharmacological immune product that would prevent and/or treat the disease and/or delay its progression by eliciting in the host an immune response that counteracts the pathological effect of the infection. A therapeutic vaccine differs from a prophylactic vaccine in that a therapeutic vaccine will induce protection in a patient who already has the infection or the disease.

In this document and in its claims, the verb "to comprise" and its conjugations is used in its non-limiting sense to mean that items following the word are included, but items not specifically mentioned are not excluded. In addition the verb "to consist" may be replaced by "to consist essentially of" meaning that a product or a composition or a preservation mixture as defined herein may comprise additional component(s) than the ones specifically identified, said additional component(s) not altering the unique characteristic of the invention.

In addition, reference to an element by the indefinite article "a" or "an" does not exclude the possibility that more than one of the element is present, unless the context clearly requires that there be one and only one of the elements. The indefinite article "a" or "an" thus usually means "at least one".

All patent and literature references cited in the present specification are hereby incorporated by reference in their entirety.

The invention is further illustrated by the following example, which should not be construed for limiting the scope of the present invention.

### Description of the figures

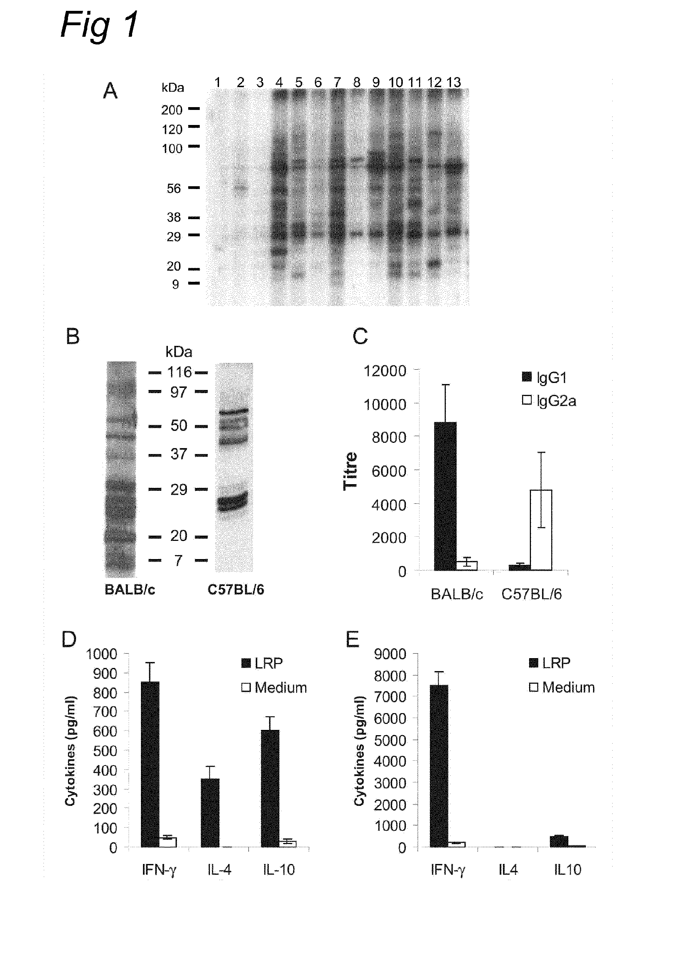

**Fig. 1****.** (A) *L. infantum* ribosomal proteins were electrophoresed on linear 10-14% gradient SDS-PAGE gel, transferred onto nitrocellulose blots and incubated with the sera of healthy dogs (lanes 1-3), and sera from dogs suffering VCL (lanes 4-13). Sera were employed at a 1/200 dilution. As secondary reagent a horseadish peroxidase conjugated anti-dog IgG antibody was used (B-E). Four BALB/c mice were s.c. infected 5 × 10⁴ *L. major* stationary-phase promastigotes in the left footpad and sera were obtained eight weeks after challenge. Four C57BL/6 mice were i.d. infected with 300 metacyclic promastigotes of *L. major* into the ear dermis and sera were obtained at week 14 postchallenge. Pre-infection sera were also obtained for both strains before parasite challenge.

(B) *L. major* ribosomal proteins were electrophoresed on linear 10-14% gradient SDS-PAGE gel transferred onto nitrocellulose blots and incubated with the pooled sera from the BALB/c or the C57BL/6 infected mice. Sera were employed at a 1/200 dilution. None of the pre-infection sera showed reactivity against LRP (not shown). (C) Titres for IgG1 and IgG2a antibodies against LRP in both mice strains were determined individually by ELISA. (D) Eight weeks after infection BALB/c mice were euthanized and their popliteal DLN cells were cultured in vitro for 48 h in the presence of 12 µg ml-1 ofL. major LRP or in medium alone. The levels of IFN-γ, IL-4 and IL-10 were assessed by ELISA in the culture supernatants. (E) Fourteen weeks after infection C57BL/6 mice were euthanized and their retromaxilar DLN were treated as in D.

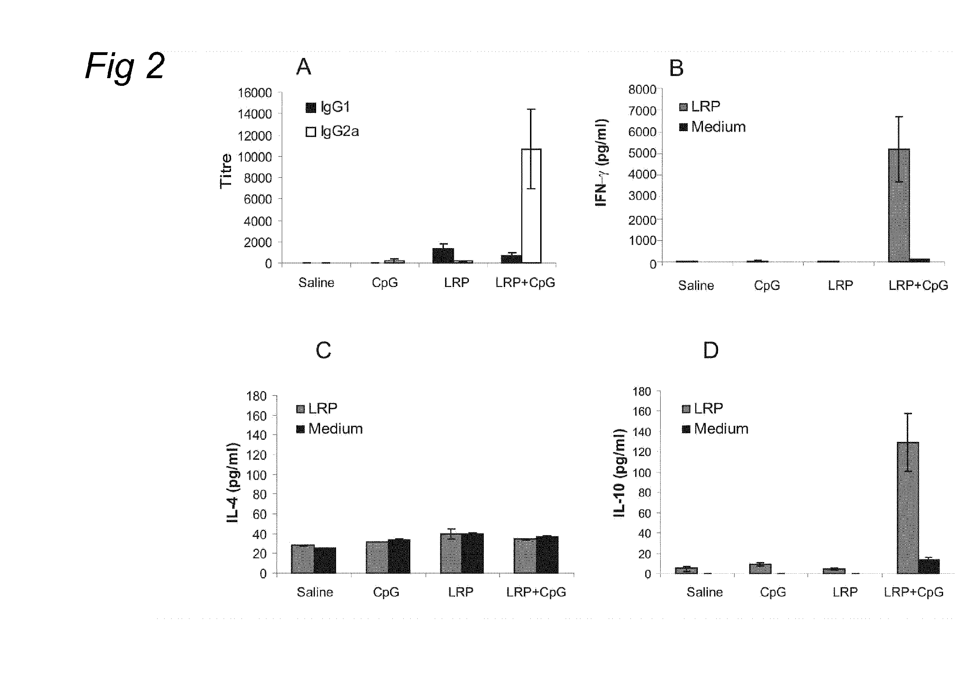

**Fig. 2****.** (A) Analysis of the specific humoral response induced in BALB/c mice. BALB/c mice (six per group) were s.c. immunized in the right footpad with three doses of the *L. major* ribosomal proteins alone (LRP), or adjuvated with CpG ODN (LRP + CpG), with the CpG ODN adjuvant alone (CpG) or with PBS (saline). Four weeks after the third immunization, mice were bled and sera were assessed by ELISA for specific anti-LRP antibody responses of both IgG1 (black bars) and IgG2a (white bars) isotype. None of the pre-immune sera showed reactivity. (B-D) Four weeks after vaccination mice were euthanized and their spleens were obtained and cultured in vitro for 48 h in the presence of LRP (grey bars) or medium alone (black bars). The levels of IFN-γ (B), IL-4 (C) and IL-10 (D) were assessed by ELISA in the culture supernatants.

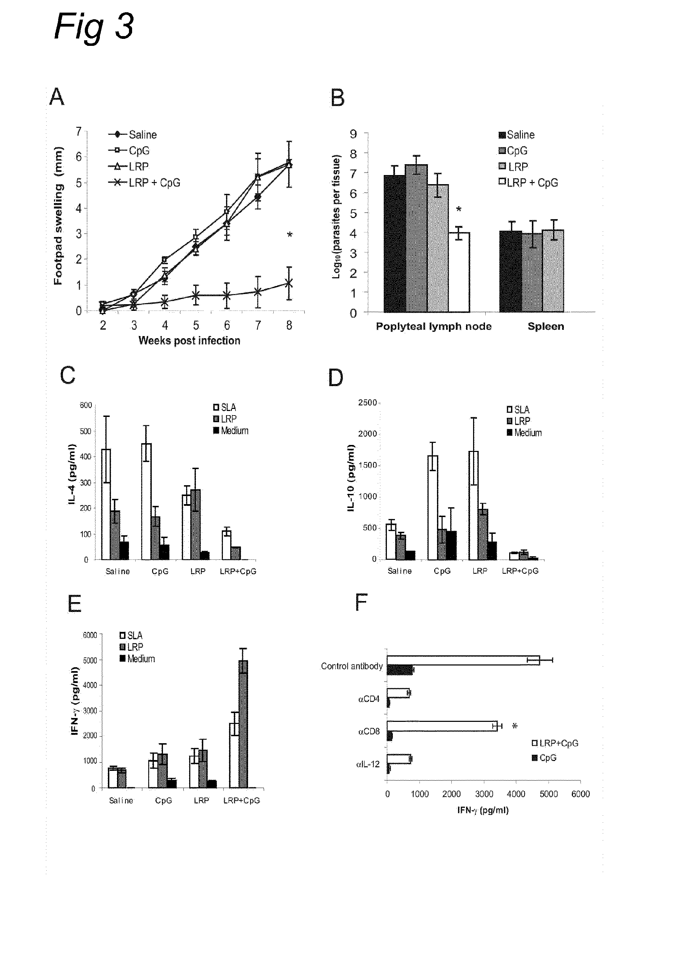

**Fig. 3****.** (A) Course of *L. major* infection in BALB/c vaccinated mice. Mice (six per group) were s.c. immunized as indicated in Fig. 2. One month after the last immunization, the animals were infected in the left hind footpad with 5 × 10⁴ *L. major* stationary-phase promastigotes. Footpad swelling is given as the difference of thickness between the infected and the uninfected contralateral footpad. Results represent the mean and standard deviation for two independent experiments. * P< 0.001 significant differences in inflammation for LRP + CpG ODN vaccinated mice versus the CpG ODN mice group at week eight post-challenge. (B) At week eight after infection the number of viable parasites in the popliteal DLN of the infected leg and spleen were individually determined by limiting dilution. Results represent the mean and standard deviation for two independent experiments. * P< 0.01 significant differences in popliteal parasite burden for LRP + CpG ODN vaccinated mice versus the CpG ODN mice group at week 8 post-challenge. (C-D) Cytokine production in vaccinated and infected mice was determined eight weeks after *L. major* challenge. Popliteal DLN of the infected leg were obtained an cultured in vitro for 48 h in the presence of SLA (white bars), LRP (grey bars) or medium alone (black bars). Levels of IL-4 (C), IL-10 (D), and IFN-γ (E) in culture supernatants were tested by ELISA. This experiment was repeated with similar results. (F) Analysis of the involvement of IL-12 and T cells in the production of IFNγ associated with the protection conferred by vaccination with LRP + CpG ODN. Popliteal LN from mice vaccinated with CpG ODN (black bars) and LRP + CpG ODN (white bars) were obtained eight weeks after challenge with 5 × 10⁴ *L. major* stationary phase promastigotes and culture-stimulated with LRP in the presence of either anti-IL-12, anti-CD4 or anti-CD8, and control monoclonal antibodies. The levels of IFN-γ were assessed by ELISA after 78 h of incubation. Differences in IFN-γ production between treatment with anti-CD8 monoclonal antibodies and treatment with control antibodies were statistically significant (* P < 0.05). Data correspond to one representative experiment of two independent experiments with similar results.

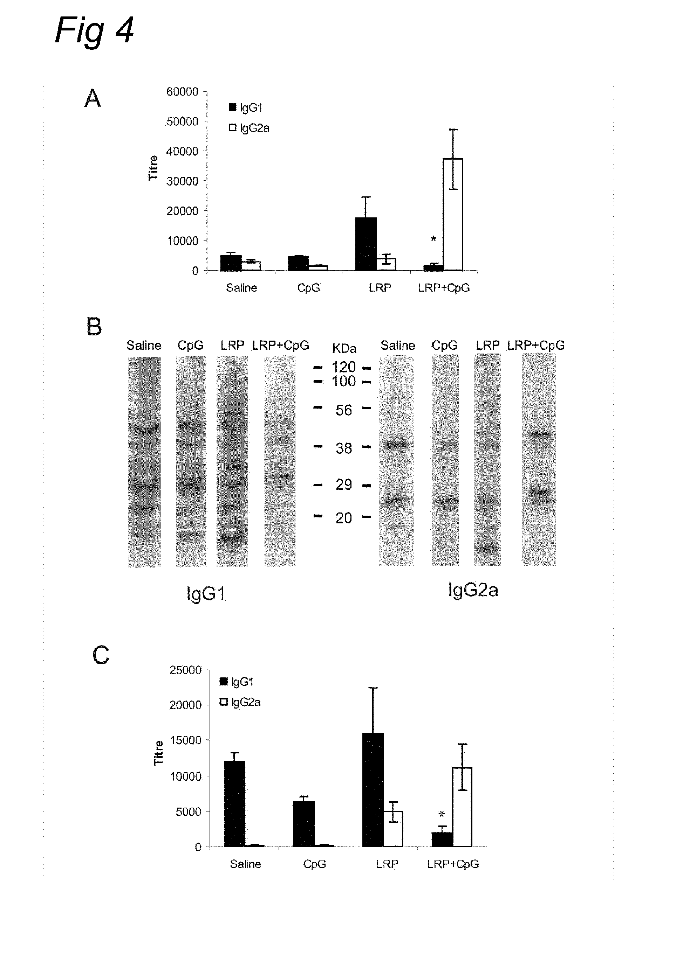

**Fig. 4****.** Analysis of the IgG1/IgG2a polarization. (A) Serum samples were obtained eight weeks after challenge and the titre for IgG1 and IgG2a antibodies against LRP were determined individually by ELISA. Differences in the IgG1 titre between mice vaccinated with LRP + CpG ODN and the other three groups were statistically significant (* P < 0.01). (B) *L. major* LRP were resolved on linear 10-14% gradient SDS-PAGE gel transferred onto nitrocellulose blots and incubated with the pooled sera from the indicated mice groups a 1/200 dilution. Antibody responses of both IgG1 and IgG2a isotype are shown. (C) The same sera were employed for the determination of the IgG1 and IgG2a titres against SLA. Differences in the IgG1 titre between mice vaccinated with LRP + CpG ODN and the other three groups were statistically significant (* P < 0.02).

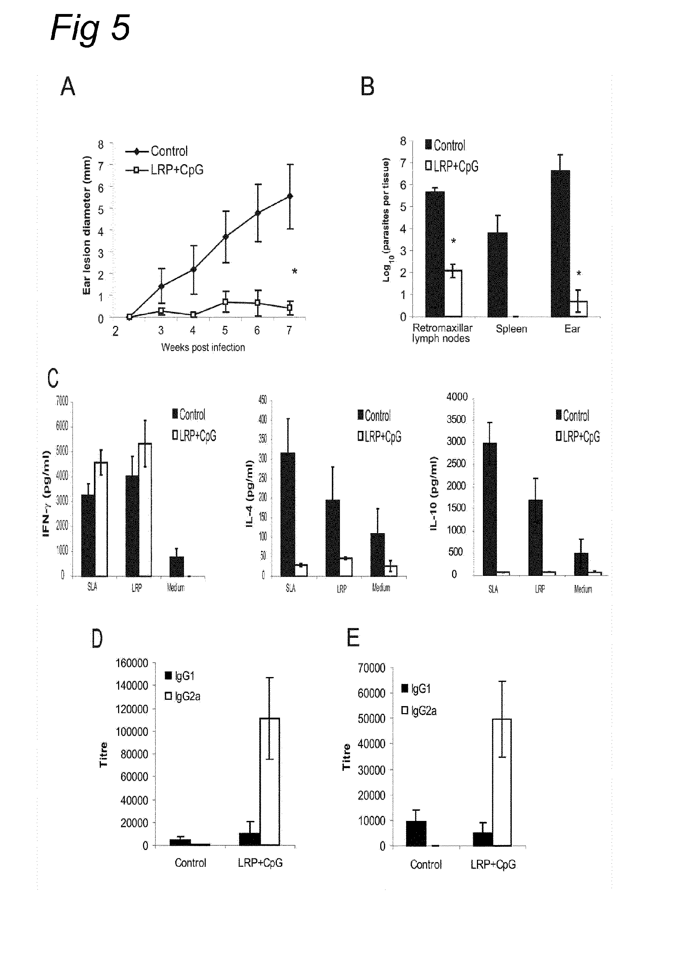

**Fig. 5****.** Six BALB/c mice were vaccinated with LRP + CpG ODN and infected in the left footpad as indicated in Fig. 3. Eighteen weeks after the first parasite challenge mice were i.d. re-infected in the ear with 300 metacyclic promastigotes of *L. major.* As control six naive mice were also i.d. challenged in the ear . (A) Course of *L. major* infection in protected and re-infected BALB/c mice. Values represent the mean lesion diameter + standard deviation (SD). * P< 0.0001 significant differences in inflammation for reinfected versus control infected mice at week 7 post-challenge. (B) Seven weeks after re-infection, mice were euthanized and parasite burden in the ear dermis, spleen and in the local DLN was individually quantitated. Results are expressed as the mean ± SD of twelve ears and DLN. * P < 0.001 significant decrease for re-infected versus infected control mice. (C) After euthanization the retromaxilar DLN from control (black bars) and re-infected mice (white bars) were obtained an cultured in vitro for 48 h in the presence of SLA, LRP or medium alone. The levels of IFN-γ, IL-4 and IL-10 were assessed by ELISA in the culture supernatants. (D-E) Serum samples were obtained seven weeks after re-challenge and the titre for IgG1 and IgG2a antibodies against LRP (D) and SLA (E) was determined individually by ELISA.

**Fig. 6****.** Protection against *L. major* infection in C57BL/6 mice. Mice (six per group) were s.c. immunized in the right footpad with three doses of LRP + CpG ODN and with CpG ODN alone (A) IFN-γ, IL-4 and IL-10 production by splenocytes of C57BL76 vaccinated mice. Four weeks after vaccination with CpG ODN (black bars) or LRP + CpG ODN (white bars) mice were euthanized and their spleens were obtained and cultured in vitro for 48 h in the presence of LRP or medium alone. The level of cytokines was assessed by ELISA in the cultures supernatant. (B) Course of *L. major* infection in C57BL/6 vaccinated mice. Twelve mice per group were immunized as described above and four weeks after the last inoculation they were infected by i.d. inoculation into the ear with 300 metacyclic promastigotes of *L. major.* Values represent the mean lesion diameter + standard deviation (SD). * P < 0.001 significant decrease in the inflammation between the two mice groups. (C) Parasite burden in the ear dermis and in the local DLN from mice vaccinated with CpG ODN (black bars) or LRP + CpG ODN (white bars) quantitated at weeks five (six mice per group) and thirteen (six mice per group) postinfection. Results are expressed as the mean ± SD of twelve ears and DLN. * P < 0.01 significant decrease between both mice groups. (D-E) Production of IFN-γ in mice vaccinated with CpG (black bars) or LRP + CpG ODN (white bars). Retromaxillar cells were obtained 5 weeks after infection and stimulate in culture in the presence of SLA, LRP and medium alone (D) or with LRP in the presence of anti-IL-12, anti-CD4 or antiCD8, and control monoclonal antibodies. The level of IFN-γ was assessed by ELISA after 78 h of incubation. Differences in IFN-γ production between treatment with antiCD8 monoclonal antibodies and treatment with control antibodies were statistically significant (* P < 0.01). (F) Serum samples were obtained at week 5 and 13 after challenge and the titres for IgG1 and IgG2a antibodies against LRP were determined individually by ELISA. (G) Production of IL-10 in mice vaccinated with CpG ODN (black bars) or LRP + CpG ODN (white bars). Retromaxillar cells were obtained 5 weeks after infection and stimulate in culture in the presence of SLA, LRP or medium alone. The level of IL-10 was assessed by ELISA after 78 h of incubation.

### Examples

### MATERIALS AND METHODS

### Mouse strains and parasites.

Female BALB/c mice were 6-8 week old at the onset of experiments and were purchased from Harlan Interfauna Ibérica S.A. (Barcelona, Spain). *L. major* parasites (clone WHOM/IR/-173) and clone V1 (MHOM/IL/80(Friedlin) were kept in a virulent state by passage in BALB/c mice. *L. major* amastigotes were obtained from popliteal draining lymph nodes (DLN) and transformed to promastigote by culturing at 26 °C in Schneider's medium (Gibco, BRL) supplemented with 20% foetal calf serum (FCS) until they reached the late stationary phase. Promastigotes of both clones were cultured at 26 °C in Schneider's medium (Gibco, BRL) supplemented with 20% FCS. Infective-stage promastigotes (metacyclics) of *L. major* (clone V1) were isolated from stationary cultures by negative selection using peanut agglutinin (Vector Laboratories, Burlingame, CA.). *L. infantum* (MCAN/ES/96/BCN/150, MON-1) promastigotes were cultured at 26 °C in RPMI medium (Gibco, BRL) supplemented with 10% FCS.

### CpG ODN and leishmanial antigens.

For preparation of *Leishmania* ribosomal protein extracts (LRP), *L. major* and *L*. *infantum* promastigotes were harvested, washed twice in pre-chilled PBS and resuspended in 1 ml NP40 (Roche Diagnostics, GmbH, Manheim Germany, cat. N. 11332473001) lysis buffer (10 mM Tris ClH pH 8.0, 150 mM NaCl, 1.5 mM MgCl2 and 0.5 % NP40, PMSF 1mM, Leopeptin 8 µg/ml, Aprotinin 4 µg/ml and Pentatin 8 µg/ml), and pipetted up and down 10 times. After lyses, samples were micro fuged at 3,000 × g for 2 min at 4 °C to pellet the nuclei. Supernatant was twice microfuged at 13,000 × g for 15 min at 4 °C and the ribosomes were prepared from the cytosolic supernatant as described in (45). Briefly, cytosol was submitted to high speed centrifugation at 90,000 rpm for 30 min at 4 °C in a Beckman TL100.3 rotor. The crude ribosomal pellet was resuspended in buffer A (20 mM Tris-HCl, pH 7.4, 500 mM AcNH4, 100 mMMgCL2, 5mM β-mercaptoethanol) and centrifuged through a discontinuous sucrose gradient (20/40%) in buffer A at 90,000 rpm at 4 °C in a TL100.3 rotor. The pellet of washed ribosomes was dissolved in PBS, sonicated and stored at - 70 °C.

Total proteins of *L. major* (soluble *Leishmania* antigen [SLA]) was prepared by three freezing and thawing cycles of stationary promastigotes of *L. major* suspended in PBS. After cell lysis, soluble antigens were separated from the insoluble fraction by centrifugation for 15 min at 12,000 g using a microfuge and stored at - 70 °C. Phosphorothioate-modified ODN sequences containing CpG motifs (CpG ODN) were synthesized by Isogen (The Netherlands). The sequences of the immunostimulatory ODN (5' to 3') were TCAACGTTGA and GCTAGCGTTAGCGT.

### Immunizations and parasite challenge.

BALB/c mice were subcutaneously (s.c.) inoculated in the right footpad with either 12 µg of *L. major* LRP alone or plus 50 µg of CpG ODN (25 µg of each immunostimulatory ODN), CpG ODN (50 µg) adjuvant alone, or phosphate saline buffer (PBS). Each group was boosted 2 and 4 weeks later using the same regime. First parasite challenge was carried out by s.c. inoculation with 5 × 10⁴ stationary-phase promastigotes of *L. major* (clone WHOM/IR/-173) into the left (untreated) footpad four weeks after the last inoculation. The progress of the infection was followed by measuring the thickness with a metric calliper. The contralateral footpad of each animal represented the control value, and the swelling was calculated as follows: thickness of the left footpad minus thickness of the right footpad. The animals were euthanized when the lesions became necrotic. For re-infection six BALB/c mice were vaccinated and infected as above. After eighteen weeks, 300 metacyclic promastigotes of *L. major* (clone V1) were injected into the dermis of both ears of each mouse. The evolution of the infection was monitored by measuring the diameter of the indurations of the ear lesions with a metric calliper. As control a group of six naive BALB/c mice were also infected in the ear dermis.

C57BL/6 mice were injected s.c. in the footpad with 12 µg of *L. major* LRP + 50 µg CpG ODN (25 µg of each immunostimulatory ODN), and 50 µg of CpG ODN (50 µg) adjuvant alone. These mice were boosted two and four weeks later with the same immunization regime. The infection was performed 4 weeks after the last vaccination by intradermal (i.d.) inoculation of 300 metacyclic promastigotes of *L. major* (clone V1) into the dermis of both ears of the mouse. The evolution of the infection was monitored by measuring the diameter of the indurations of the ear lesion with a metric calliper.

### Parasite quantization.

The number of parasites was determined in the ears by limiting dilution assay (6). Briefly, ears were recovered from infected mice. The ventral and dorsal sheets of the infected ears were separated. Ear sheets were deposited in Dulbecco's modified Eagle medium containing Liberase CI enzyme blend (50 µg ml-1). After 2 hours of incubation at 37 °C, the tissues were cut into small pieces, homogenized and filtered using a cell strainer (70 µm-pore size). The homogenized tissue was serially diluted in a 96-well flatbottomed microtiter plate containing Schneider's medium plus 20% FCS. The number of viable parasites was determined from the highest dilution at which promastigotes could be grown up to 7 days incubation at 26 °C. The number of parasites was also determined in the local draining lymph nodes (DLNs) of infected ears (retromaxilar) and footpad (popliteal) and in the spleen. Organs were recovered, mechanically dissociated and then serially diluted as above. Parasite load is expressed as the number of parasites in the whole organ.

### Measurement of cytokines in supernatants.

Spleens and the corresponding local DLNs were removed aseptically, mechanically dissociated and seeded in complete RPMI medium (RPMI 1640 supplemented with 10% FCS, 2 mM glutamine, and 10 mM 2-mercaptoethanol). 5 × 10⁶ cells ml⁻¹ were seeded in 48-well plates during 48 h at 37 °C in the presence of LRP (12 µg ml⁻¹) or SLA (12 µg ml⁻¹). The release of IFN-γ, IL-10 and IL-4 was measured in the supernatants of splenocytes and DLN cells cultures by commercial ELISA kits (Diaclone, Besançon, France). In some cases, DLN cells stimulated with 12 µg ml⁻¹ of LRP were incubated in the presence of 10 µg ml⁻¹ of monoclonal antibody (mAb) against either mouse CD4 (GK 1.5), mouse IL-12 (C17.8), mouse CD8 (53-6.7). Appropriate isotype-matched controls were also analyzed in the assay. The antibodies (no azide/low endotoxin™) were purchased from BD (PharMingen).

### Analysis of the humoral responses.

Serum samples were analysed for specific antibodies against LRP or SLA by ELISA or Western blot. Briefly, standard ELISA plates were coated overnight at room temperature with 100 µl of LRP (5 µg ml⁻¹ in PBS) or SLA (2 µg ml⁻¹ in PBS). The titre was determined by serial dilution of the sera, and was defined as the inverse of the highest serum dilution factor giving an absorbance > 0.2. The isotype-specific analyses were done with the following horseradish peroxidase-conjugated anti-mouse immunoglobulins (Nordic Immunological Laboratories, Tilburg, The Netherlands): anti-IgG1 (1/1000) and anti-IgG2a (1/500). Ortophenyle diamine dihydrochloride - OPD- (Dako, A/S, Glostrup, Denmark) was used as peroxidase substrate for ELISA assays. After 15 min, the reaction was stopped by addition of 100 µl of H₂SO₄ 1 M and the absorbance was read at 450 nm.

For Western blot analysis, *L. infantum* and *L. major* ribosomal proteins were obtained, resuspended in Laemmli's buffer, resolved by SDS-PAGE and transferred to nitrocellulose membranes (Amersham, Aylesbury, UK). The blots were probed with the sera for control dogs, dogs suffering VCL leishmaniasis, or the sera from the different groups of mice employed in this work at the indicated dilutions. As secondary antibodies horseradish peroxidase-conjugated anti-mouse anti-IgG (1/1000), anti-IgG1 (1/1000), anti-IgG2a (1/500) immunoglobulin, and anti-dog-IgG (1/2000) purchased from Nordic Immunological Laboratories (Tilburg, The Netherlands) were used.

### Statistical analysis.

Statistical analysis was performed by a Student's t-test. Differences were considered significative when P< 0.05.

### RESULTS

### Antigenicity of the LRP during infection.

In order to analyze the antigenicity of the *Leishmania* ribosomal proteins (LRP) the reactivity against *L. infantum* LRP of sera from dogs naturally infected with this parasite was assayed by Western blot. It was observed that sera from dogs suffering the active disease recognized a large number of protein bands in the LRP extract (Fig. 1A). Sera from C57BL/6 and BALB/c mice experimentally infected with *L. major* also recognize many protein bands in *L. major* LRP, being the number of ribosomal proteins recognized by the IgG antibodies present in the sera from BALB/c susceptible mice higher than the number of proteins recognized by these antibodies in the sera of the C57BL/6 resistant mice (Fig. 1B).

Since the induction of IgG1 and IgG2a antibodies can be used as a marker of Th₂-type and Th₁-type immune responses (8), we made the analysis of the IgG1/IgG2a polarization against LRP in *L. major* infected mice. In BALB/c mice the anti-LRP response was predominantly of the IgG1 isotype whereas it was of the IgG2a isotype in C57BL/6 mice (Fig. 1C). The IFN-γ, IL-4 and IL-10 production after in vitro stimulation of DLN cells with LRP in both mice strains was also determined. In BALB/c mice suffering CL the production of LRP-specific IFN-γ was detected but also the production of IL-4 and IL-10 was strongly stimulated being the IFN-γ/IL-4 ratio ≈ 2.4 and the IFNy/IL-10 ratio ≈ 1.4 (Fig. 1D). In contrast, no IL-4 (< 7.5 pg ml-1) and a high IFN-γ/IL-10 ratio (≈ 15) were obtained when the DLN cells from healed C57BL/6 mice were stimulated with LRP (Fig. 1E).

### Immunogenicity of the LRP in BALB/c mice.

The immune responses to LRP were evaluated in BALB/c mice after administration of the ribosomal proteins in the absence and in the presence of CpG ODN. After vaccination with LRPE + CpG ODN the anti-LRPE humoral response was predominantly of the IgG2a isotype, whereas lower titre of antibodies of the IgG1 isotype was detected in the sera from mice immunized with LRPE alone (Fig. 2A). After in vitro stimulation with LRPE spleen cells from mice immunized with LRPE + GpG ODN secreted higher levels of IFN-γ than those secreted by spleen cells from controls and from mice immunized with LRPE alone (Fig. 2B). No increase in IL-4 production was observed after stimulation with LRPE in any group (Fig. 2C). Remarkably, specific IL-10 was detected in the supernatant of cultures established from spleens of LRPE + CpG ODN vaccinated mice being the IFNy/IL-10 ratio ≈ 40 (Fig. 2D). Altogether, these results demonstrate that the LRPE administered without adjuvants induced only weak IgG1 humoral responses, but coadministration with CpG ODN boosted a Th₁-like response against these antigens in BALB/c mice.

### Vaccination with LRPE + CpG ODN protects BALB/c mice against a L. major challenge.

Since redirection of the Th₂ responses induced by disease associated antigens toward a Th1 response has been considered as a promising approach for the development of vaccines against *Leishmania* (7) we analyzed whether vaccination with LRPE + CpG ODN was able to induce protection against *L. major* infection. Fig. 3A shows that LRPE + CpG OND induce effective protection since the footpad swelling in these mice was reduced (mean value of 0.7 mm at week 8) when compared with that of controls and mice vaccinated with LRPE alone (mean value ≈ 5.5 mm). We then analyzed the parasite load in popliteal DLN and in the spleen of the four groups of mice. DLN from mice immunized LRPE-CpG showed a ≈ 3-log reduction in parasite burden relative to the other groups. In addition, while similar parasite loads were found in the spleen of control mice and of mice immunized with LRPE alone no parasites could be detected in the spleen of the LRPE + CpG ODN vaccinated mice (Fig. 3B).

To determine the immunological parameters associated with the LRPE + CpG ODN induced protection, the SLA or the LRPE-driven production of IL-4, IL-10 and IFN-γ was assayed. SLA or LRPE specifically-induced IL-4 and IL-10 production was detected in DLN cells from controls (saline and CpG) and from mice immunized with LRPE alone (Fig. 3C-D). In contrast, DLN cells from mice immunized with LRPE + CpG ODN produced higher amounts of IFN-γ than those detected in the other three groups (Fig. 3E). The contribution of CD4⁺ and CD8⁺ T cells and the dependence on IL-12 to the LRPE specific production of IFN-γ was also analyzed. As shown in Fig. 3F, the production of IFN-γ was completely inhibited by anti-IL-12 or anti-CD4 monoclonal antibodies. The addition of anti-CD8 antibodies to the DLN cell cultures only partially reduced the amounts of this cytokine in the supernatants.

Given that in BALB/c mice the IL-4 dependent production of high titres of antibodies is associated with disease progression we analyzed by ELISA the humoral responses elicited against the LRPE at week eight after infection. The antibodies against the LRPE elicited by the parasite challenge in mice that had been immunized with LRPE + CpG ODN were mainly of the IgG2a isotype. Also, lower titre of anti-LRPE antibodies of the IgG1 isotype were present in the sera of protected mice when compared with those immunized with LRPE alone and in the two control groups (Fig. 4 A). The reduction of the IgG1 titre against LRPE correlated to a decrease in the number of ribosomal proteins bands recognized by the IgG1 antibodies from sera of the LRPE + CpG ODN vaccinated mice. As shown in Fig. 4B, the IgG1 isotype antibodies from sera of mice immunized with saline, CpG or LRPE alone recognized a higher number of protein bands in LRPE western blots, whereas only a few bands were recognized by the IgG1 antibodies of the protected mice. The western blot analysis also showed that there was not an increase in the number of protein bands recognized by the IgG2a antibodies in the sera from LRPE + CpG ODN vaccinated mice when compared with the other three groups (Fig. 4B). Vaccination with LRPE + CpG ODN also conditioned the global anti-*Leishmania* humoral response induced by *L. major* infection. The antibodies against SLA elicited by the parasite challenge in mice that had been immunized with LRPE + CpG ODN were mainly of the IgG2a isotype being the anti-SLA titre of the IgG1 isotype antibodies significantly lower than those detected in the other three groups (Fig. 4C).

### LRPE + CpG ODN vaccinated and infected mice are resistant against L. major re-infection in the ear dermis.

To determine whether LRPE + CpG ODN vaccinated and infected mice were able to control a second parasite challenge six BALB/c mice were vaccinated and infected in the footpad as described above. The footpad swelling of these mice group were < 0.7 mm during 18 weeks (data not shown). Then, these protected mice were re-infected with 300 *L. major* metacyclic promastigotes in the ear dermis. A control group of six naive mice was also infected. It was observed that the LRPE + CpG ODN vaccinated re-infected mice were protected against the development of dermal lesions since no pathology was observed in these mice whereas the control mice developed patent ear lesions at week seven (Fig. 5A). The parasite load in the ear dermis and in the retromaxillar DLN was also significantly different between the two groups (Fig. 5B). The low parasite load in the ear and in the DLN of vaccinated re-infected mice correlates to the absence of parasite in the spleen. In order to know the cellular response occurring after re-infection the secretion of IFN-γ, IL-4, and IL-10 by retromaxillar DLN cells after *in vitro* stimulation with LRPE or SLA was analyzed (Fig. 5C). In control mice, as also occur when mice were infected in the footpad (Fig. 3C-E) a specific production of IFN-γ were detected, but also the IL-4 and IL-10 production was strongly stimulated. In contrast, vaccinated-reinfected mice DLN cells produced high amounts of specific IFN-γ, being the IL-4 and IL-10 barely undetected (Fig. 5C). In agreement, their IgG humoral response against LRPE (Fig. 5D) and SLA (Fig. 5E) was of the IgG2a isotype.

### Vaccination with LRPE + CpG ODN confers protection against dermal pathology due to L. major challenge in C57BL/6 mice.

Given that vaccination with LRPE + CpG ODN protects against *L. major* infection in BALB/c mice by the redirection the Th₂ immune response against LRPE towards a Th₁ response we analyzed the effect of the administration of this vaccine in C57BL/6 mice, a model that naturally develop Th₁ responses against *Leishmania* antigens. A group of C57BL/6 mice was immunized with three doses of the LRPE + CpG ODN and control mice received only the CpG ODN adjuvant. Inoculation induced a Th₁ response demonstrated by the in vitro production of IFN-γ in the supernatant of spleen cells cultures stimulated with LRPE. The presence of specific IL-10 was also detected in the supernatant of spleen cell cultures established from LRPE + CpG ODN vaccinated mice, whereas no specific IL-4 production was observed after stimulation (Fig. 6A). LRPE + CpG ODN vaccinated mice were protected against the development of dermal lesions since little or no pathology was observed (Fig. 6B). CpG ODN immunized mice developed lesions that reached a peak at week seven and were almost completely healed at week 13. Since in this model the number of parasites in the infected site peak just before the development of lesion (5), we determined the parasite load in the ear and in the local DLN (retromaxilar) at week five. The number of parasites in the ear dermis of vaccinated mice had a ≈ 300-fold reduction (1.0 × 10⁴ parasites for LRPE + CpG ODN and 3 × 10⁶ parasites for CpG-ODN immunized mice) and ≈ 40-fold reduction in the DLN (5.0 × 10⁴ parasites for LRPE + CpG ODN and 2 × 10⁶ parasites for CpG ODN mice). After healing, in control mice (13 weeks after challenge) a reduction in the number of parasites was observed in all of the groups.

To determine the immunological parameters associated with protection, the antigen driven production of IL-4, IL-10 and IFN-γ was assayed. At week five after challenge cultures of the DLN cells were established and stimulated with LRPE or SLA. As shown in Fig. 6D the cells from vaccinated mice produced more SLA and LRPE specific IFN-γ than those from control mice. The contribution of CD4⁺ and CD8⁺ T cells and the dependence on IL-12 to the LRPE specific production of IFN-γ was also analyzed. The secretion of IFN-γ was completely inhibited by anti-IL-12 or anti-CD4 monoclonal antibodies. The anti-CD8 antibodies treatment only partially reduced the level of this cytokine. These data demonstrated that in C57BL/6, vaccination with LRPE + CpG ODN induced a reduction in pathology and in the parasite burden in the skin and in the local DLN that was correlated to the induction of an earlier specific Th₁ response against LRPE. In agreement, IgG2a specific anti-LRPE antibodies were detected earlier and with higher titres in the vaccinated versus the control mice (Fig. 6F). We also detected a SLA or LRPE antigen-specific production of IL-10 that was not statistically different between vaccinated and control mice.

### DISCUSSION

In this work we have shown that antibodies reacting with many of the parasite ribosomal proteins are observed in the sera from dogs with VCL, and in mice infected with *L. major,* indicating that they are strong antigens during *Leishmania* natural and experimental infections. The response of infected BALB/c mice against LRPE was of the Th₂ type since anti-LRPE antibody response was predominantly of the IgG1 isotype. This observation was reinforced by the fact that after *in vitro* stimulation of the DLN cells from infected mice with LRPE, high amounts of IL-4 were detected in the culture supernatants. Although LRPE were also implicated in the in vitro production of INF-γ, comparable levels of IL-10 were detected, a pleiotropic anti-inflammatory cytokine that renders infected macrophages unresponsive to the activation signals for parasite destruction (31). Furthermore, given that the effects of IL-4 and IL-10 in promoting disease in BALB/c mice appear to be additive (reviewed in (37)), stimulation of these cytokines by LRPE can be taken as an indication that the host responses against ribosomal antigens favour parasite expansion and persistence in the BALB/c mice. We may assume that early after *Leishmania* infection, the immune system of the host is primed by the high abundant LRPE released by parasite cytolysis that afterwards, may be boosted as a result of parasite proliferation. Thus, the strong immunogenicity of LRPE and their pathoantigenic role probably relies on their high abundance and antigenic specificity (in spite of their evolutionary conserved character). On the contrary, the C57BL/6 mice responses against LRPE were of the Th₁ type, with the generation of IgG2a specific antibodies and the production *in vitro* of high levels specific INF-γ, being the IL-4 levels undetectable. Also, we found a LRPE specific production of IL-10 at week 14 post-infection. Given that the IFN-γ/IL-10 ratio was similar to that obtained when cells were stimulated with SLA (data not shown) we suggest that IL-10 production induced by these antigens might be associated with the regulatory response that favours the persistence of the parasite that has been seen in this model (4). Our data indicate that the response against LRPE are in agreement with the bias toward the induction of Th₂-mediated humoral responses related to pathology in susceptible host and Th₁-associated protective responses in resistant host.

Since redirection of the Th₂ responses induced against some *Leishmania* epitopes towards a Th₁ response is likely to be a promising strategy to induce protection against *L. major* infection (7) we first decided to analyze in BALB/c mice the immunogenicity of the LRPE co-administered with CpG ODN, an adjuvant that confers a Th₁-related long term immunity and protection when immunize with different *leishmanial* antigens (43) and that also can suppress some parasite specific Th₂ responses in mouse (12, 57). As shown in Fig. 2, the immune response developed in BALB/c was found to be of the Th₁ type since immunized mice developed anti-LRPE antibodies of the IgG2a isotype and splenocytes from vaccinated mice produced high amounts of IFN-γ, but not IL-4, after in vitro stimulation with LRPE. Similar specific immune responses were generated in C57BL/6 mice when immunized with the LRPE + CpG ODN (Fig. 6A). In both mouse strains, the LRPE-driven production of IL-10 was also observed after immunization.

Although it has been reported that vaccination with genetic vaccines coding for L31 can be implicated in the production of specific IL-10 after genetic vaccination (44), we believe that the IL-10 production might be more related to the homeostatic control of the Th₁ responses occurring after administration of LRPE + CpG ODN. In fact, it has been recently reported that the INF-γ producing Th₁ cells can also been implicated in the production of IL-10 as a mechanism of feedback control (2). Moreover, the high IFNy/IL-10 ratio values obtained might provide a good prediction of vaccine outcome (44).

Data presented here indicated that the co-administration of LRPE + CpG ODN induce protection in two different models of cutaneous experimental leishmaniasis: high dose inocula in the footpad of BALB/c mice (widely used in cutaneous leishmaniasis vaccine assays), and low dose in the ear of C57BL/6 mice (a model that more closely mimics the human disease in terms of route and infectious dose). Vaccinated BALB/c mice had a reduced parasite burden in the popliteal DLN with the absence of parasite dissemination in the spleen. Also very low inflammation was detected in the infected footpads. Vaccinated C57BL/6 mice were protected against ear dermal pathology showing and a reduction in the parasite burden in the skin and in the DLN. This protection is comparable to that displayed by C57BL/6 mice vaccinated with heat-killed *Leishmania* antigen plus CpG ODN and a multicomponent vaccine composed of LACK, LmSTI1 and TSA also tested in this model (28, 29, 43).

Importantly, protection in both strains is correlated to the generation of Th₁ specific immune responses against LRPE. The *in vitro* analysis of the cellular responses was measured at week 8 after infection in BALB/c mice, and at week 5 (coinciding with the peak in the parasite load (4)) in C57BL/6 mice. DLN cells from both strains of mice vaccinated with LRPE + CpG ODN secreted higher levels of IFN-γ than their corresponding control groups when stimulated with the LRPE. The IFN-γ response was found to be IL-12 dependent and produced by CD4+ T cell with a lesser contribution for CD8+ T cells. As expected, the SLA-specific production of IFN-γ was higher for the protected mice in both strains. In BALB/c mice, generation of the Th₁ responses in the protected mice correlates to the generation of predominant IgG2a specific antibodies against LRPE (Fig. 4A). Some differences between strains were observed in the *in vitro* production of IL-10. Our data showed that protected BALB/c mice produced significantly lower levels of IL-10 than controls after *in vitro* stimulation with both LRPE and SLA, in agreement with the implication of this cytokine with the susceptibility in this model (35). On the other hand, both control and vaccinated C57BL/6 LNC produced IL-10 after in vitro stimulation with LRPE or SLA during the acute phase of infection (Fig. 6F). As occurred for IFN-γ, IL-10 levels were higher when cell were stimulated with LRPE than with SLA. The fact that IFN-γ and IL-10 production followed a similar profile, can be taken as an indication that IL-10 production after vaccination is reflecting the homeostatic mechanisms that controls the harmful effects that a strong Th₁ will cause in the host (4). Remarkably, we have found that protection showed in the BALB/c mice after vaccination with LRPE + CpG ODN is also related to a significant reduction in the production of antigen driven IL-4 after stimulation *in vitro* with LRPE or SLA. These cellular responses correlated in vivo with the reversion of the Th₂-mediated antibody responses against ribosomal proteins. Thus, sera from protected BALB/c mice presented a significant decrease in the titre and, notably, in the number of antigens recognized by IgG1 antibodies specific for LRPE. In addition, immunization of BALB/c mice with LRPE + CpG ODN also had a clear effect on the global humoral response elicited in mice by the *L. major* infection (Fig. 5C). Thus, the infection of vaccinated mice induces limited IgG1 anti-*Leishmania* specific antibodies, whereas the humoral response induced in the other assayed control mice groups was higher and with a predominance of antibodies of the Th₂ type (i.e., IgG1 isotype). As a whole, protection observed in vaccinated mice from both strains mice is correlated to the generation of Th₁ responses against LRPE that also result in the down-regulation of the IL-4 driven Th₂ and IL-10 responses against SLA in BALB/c mice.

Our data indicate that protected BALB/c mice have acquired an immunological status which conferred them the capacity to resist a further infection (an appealing feature for a vaccine that might be employed in endemic areas, where re-exposure to the parasite would be very frequent). After re-challenge in the ear dermis these mice showed a robust protection against *L. major* infection. Very low dermal lesions development (in some cases a complete absence of dermal lesions was detected) and a substantial reduction in the parasite number in the infected ear and in the DLN were found. Although DLN cells from protected mice did not produce larger quantities of IFN-γ than controls (measured at week 7 post-infection), the titre of the IgG2a antibodies against LRPE and SLA can be taken as an indication that these mice mounted a specific Th₁ protective response after the secondary challenge. Remarkably, the specific production of the IL-4 and IL-10 disease associated cytokines in these mice was very low. These data indicate that the immune state generated after the first parasite challenge is extremely potent, leading to a rapid and efficient elimination of the parasite from the site of re-infection.

Data presented here demonstrate that vaccination with LRPE + CpG ODN has direct influences on decisions of the immune system at the time of *Leishmania* infection in both, resistant and susceptible mice. In our opinion, generation of vaccines against such a complex parasite as *Leishmania,* would be optimized by incorporating different target antigens in the vaccine formulation, taking advantage of these antigens that induce the required immunity (mainly CD4⁺ and CD8⁺ IFN-γ mediated responses), and redirecting towards a Th₁ bias the pathoantigenic-driven immune responses that result in pathology (IL-4 Th₂-driven and IL-10 deactivating responses). Notwithstanding, it should be taking into account that the Th₂-response against some of these antigens may not be redirected by the usual Th₁-inducers, as occur with the meta 1 antigen of *L. major* (50).

### REFERENCES

1.Aguilar-Be, I., R. da Silva Zardo, E. Paraguai de Souza, G. P. Borja-Cabrera, M. Rosado-Vallado, M. Mut-Martin, R. Garcia-Miss Mdel, C. B. Palatnik de Sousa, and E. Dumonteil. 2005. Cross-protective efficacy of a prophylactic Leishmania donovani DNA vaccine against visceral and cutaneous murine leishmaniasis. Infect Immun 73:812-9.
2.Anderson, C. F., M. Oukka, V. J. Kuchroo, and D. Sacks. 2007. CD4(+)CD25(-)Foxp3(-) Th1 cells are the source of IL-10-mediated immune suppression in chronic cutaneous leishmaniasis. J Exp Med 204:285-97.
3.Badaro, R., D. Benson, M. C. Eulalio, M. Freire, S. Cunha, E. M. Netto, D. Pedral-Sampaio, C. Madureira, J. M. Bums, R. L. Houghton, J. R. David, and S. G. Reed. 1996. rK39: a cloned antigen of Leishmania chagasi that predicts active visceral leishmaniasis. J Infect Dis 173:758-61.
4.Belkaid, Y., K. F. Hoffmann, S. Mendez, S. Kamhawi, M. C. Udey, T. A. Wynn, and D. L. Sacks. 2001. The role of interleukin (IL)-10 in the persistence of Leishmania major in the skin after healing and the therapeutic potential of antiIL-10 receptor antibody for sterile cure. J Exp Med 194:1497-506.
5.Belkaid, Y., S. Kamhawi, G. Modi, J. Valenzuela, N. Noben-Trauth, E. Rowton, J. Ribeiro, and D. L. Sacks. 1998. Development of a natural model of cutaneous leishmaniasis: powerful effects of vector saliva and saliva preexposure on the long-term outcome of Leishmania major infection in the mouse ear dermis. J Exp Med 188:1941-53.
6. Buffet, P. A., A. Sulahian, Y. J. Garin, N. Nassar, and F. Derouin. 1995. Culture microtitration: a sensitive method for quantifying Leishmania infantum in tissues of infected mice. Antimicrob Agents Chemother 39:2167-8.
7. Campos-Neto, A. 2005. What about Th1/Th2 in cutaneous leishmaniasis vaccine discovery? Braz J Med Biol Res 38:979-84.
8. Coffman, R. L. 1993. Mechanisms of helper T-cell regulation of B-cell activity. Ann N Y Acad Sci 681:25-8.
9. Coler, R. N., and S. G. Reed. 2005. Second-generation vaccines against leishmaniasis. Trends Parasitol 21:244-9.
10. Cordeiro-Da-Silva, A., M. C. Borges, E. Guilvard, and A. Ouaissi. 2001. Dual role of the Leishmania major ribosomal protein S3a homologue in regulation of T- and B-cell activation. Infect Immun 69:6588-96.
11. Chenik, M., H. Louzir, H. Ksontini, A. Dilou, I. Abdmouleh, and K. Dellagi. 2006. Vaccination with the divergent portion of the protein histone H2B of Leishmania protects susceptible BALB/c mice against a virulent challenge with Leishmania major. Vaccine 24:2521-9.
12. Chiaramonte, M. G., M. Hesse, A. W. Cheever, and T. A. Wynn. 2000. CpG oligonucleotides can prophylactically immunize against Th2-mediated schistosome egg-induced pathology by an IL-12-independent mechanism. J Immunol 164:973-85.
13. Garcia-Alonso, M., A. Blanco, D. Reina, F. J. Serrano, C. Alonso, and C. G. Nieto. 1996. Immunopathology of the uveitis in canine leishmaniasis. Parasite Immunol 18:617-23.
14. Garcia-Alonso, M., C. G. Nieto, A. Blanco, J. M. Requena, C. Alonso, and I. Navarrete. 1996. Presence of antibodies in the aqueous humour and cerebrospinal fluid during Leishmania infections in dogs. Pathological features at the central nervous system. Parasite Immunol 18:539-46.
15. Gradoni, L. 2001. An update on antileishmanial vaccine candidates and prospects for a canine Leishmania vaccine. Vet Parasitol 100:87-103.
16. Gramiccia, M., and L. Gradoni. 2005. The current status of zoonotic leishmaniases and approaches to disease control. Int J Parasitol 35:1169-80.
17. Handman, E., A. H. Noormohammadi, J. M. Curtis, T. Baldwin, and A. Sjolander. 2000. Therapy of murine cutaneous leishmaniasis by DNA vaccination. Vaccine 18:3011-7.
18. Herwaldt, B. L. 1999. Leishmaniasis. Lancet 354:1191-9.
20. Iborra, S., M. Soto, J. Carrion, C. Alonso, and J. M. Requena. 2004. Vaccination with a plasmid DNA cocktail encoding the nucleosomal histones of Leishmania confers protection against murine cutaneous leishmaniosis. Vaccine 22:3865-76.
22. Jaafari, M. R., A. Ghafarian, A. Farrokh-Gisour, A. Samiei, M. T. Kheiri, F. Mahboudi, F. Barkhordari, A. Khamesipour, and W. R. McMaster. 2006. Immune response and protection assay of recombinant major surface glycoprotein of Leishmania (rgp63) reconstituted with liposomes in BALB/c mice. Vaccine 24:5708-17.
23. Lopez, R., R. Lucena, M. Novales, P. J. Ginel, E. Martin, and J. M. Molleda. 1996. Circulating immune complexes and renal function in canine leishmaniasis. Zentralbl Veterinarmed B 43:469-74.
24. Mancianti, F., A. Poli, and A. Bionda. 1989. Analysis of renal immune-deposits in canine leishmaniasis. Preliminary results. Parassitologia 31:213-30.
25. Martins, D. R., S. M. Jeronimo, J. E. Donelson, and M. E. Wilson. 2006. Leishmania chagasi T-cell antigens identified through a double library screen. Infect Immun 74:6940-8.
26. McMahon-Pratt, D., and J. Alexander. 2004. Does the Leishmania major paradigm of pathogenesis and protection hold for New World cutaneous leishmaniases or the visceral disease? Immunol Rev 201:206-24.
28. Mendez, S., Y. Belkaid, R. A. Seder, and D. Sacks. 2002. Optimization of DNA vaccination against cutaneous leishmaniasis. Vaccine 20:3702-8.
29. Mendez, S., S. Gurunathan, S. Kamhawi, Y. Belkaid, M. A. Moga, Y. A. Skeiky, A. Campos-Neto, S. Reed, R. A. Seder, and D. Sacks. 2001. The potency and durability of DNA- and protein-based vaccines against Leishmania major evaluated using low-dose, intradermal challenge. J Immunol 166:5122-8.
30. Miles, S. A., S. M. Conrad, R. G. Alves, S. M. Jeronimo, and D. M. Mosser. 2005. A role for IgG immune complexes during infection with the intracellular pathogen Leishmania. J Exp Med 201:747-54.
31. Moore, K. W., R. de Waal Malefyt, R. L. Coffman, and A. O'Garra. 2001. Interleukin-10 and the interleukin-10 receptor. Annu Rev Immunol 19:683-765.
32. Mougneau, E., F. Altare, A. E. Wakil, S. Zheng, T. Coppola, Z. E. Wang, R. Waldmann, R. M. Locksley, and N. Glaichenhaus. 1995. Expression cloning of a protective Leishmania antigen. Science 268:563-6.
33. Nieto, C. G., R. Barrera, M. A. Habela, I. Navarrete, C. Molina, A. Jimenez, and J. L. Serrera. 1992. Changes in the plasma concentrations of lipids and lipoprotein fractions in dogs infected with Leishmania infantum. Vet Parasitol 44:175-82.
34. Nieto, C. G., I. Navarrete, M. A. Habela, F. Serrano, and E. Redondo. 1992. Pathological changes in kidneys of dogs with natural Leishmania infection. Vet Parasitol 45:33-47.
35. Noben-Trauth, N., R. Lira, H. Nagase, W. E. Paul, and D. L. Sacks. 2003. The relative contribution of IL-4 receptor signaling and IL-10 to susceptibility to Leishmania major. J Immunol 170:5152-8.
36. Pateraki, E., R. Portocala, H. Labrousse, and J. L. Guesdon. 1983. Antiactin and antitubulin antibodies in canine visceral leishmaniasis. Infect Immun 42:496-500.
37. Peters, N., and D. Sacks. 2006. Immune privilege in sites of chronic infection: Leishmania and regulatory T cells. Immunol Rev 213:159-79.
38. Pollock, K. G., K. S. McNeil, J. C. Mottram, R. E. Lyons, J. M. Brewer, P. Scott, G. H. Coombs, and J. Alexander. 2003. The Leishmania mexicana cysteine protease, CPB2.8, induces potent Th2 responses. J Immunol 170:1746-53.
39. Probst, P., E. Stromberg, H. W. Ghalib, M. Mozel, R. Badaro, S. G. Reed, and J. R. Webb. 2001. Identification and characterization of T cell-stimulating antigens from Leishmania by CD4 T cell expression cloning. J Immunol 166:498-505.
40. Rafati, S., A. Nakhaee, T. Taheri, A. Ghashghaii, A. H. Salmanian, M. Jimenez, M. Mohebali, S. Masina, and N. Fasel. 2003. Expression of cysteine proteinase type I and II of Leishmania infantum and their recognition by sera during canine and human visceral leishmaniasis. Exp Parasitol 103:143-51.
41. Rafati, S., A. H. Salmanian, T. Taheri, M. Vafa, and N. Fasel. 2001. A protective cocktail vaccine against murine cutaneous leishmaniasis with DNA encoding cysteine proteinases of Leishmania major. Vaccine 19:3369-75.
42. Requena, J. M., C. Alonso, and M. Soto. 2000. Evolutionarily conserved proteins as prominent immunogens during Leishmania infections. Parasitol Today 16:246-50.
43. Rhee, E. G., S. Mendez, J. A. Shah, C. Y. Wu, J. R. Kirman, T. N. Turon, D. F. Davey, H. Davis, D. M. Klinman, R. N. Coler, D. L. Sacks, and R. A. Seder. 2002. Vaccination with heat-killed Leishmania antigen or recombinant leishmanial protein and CpG oligodeoxynucleotides induces long-term memory CD4+ and CD8+ T cell responses and protection against Leishmania major infection. J Exp Med 195:1565-73.
44. Roberts, M. T., C. B. Stober, A. N. McKenzie, and J. M. Blackwell. 2005. Interleukin-4 (IL-4) and IL-10 collude in vaccine failure for novel exacerbatory antigens in murine Leishmania major infection. Infect Immun 73:7620-8.
45. Rodriguez-Gabriel, M. A., M. Remacha, and J. P. Ballesta. 2000. The RNA interacting domain but not the protein interacting domain is highly conserved in ribosomal protein P0. J Biol Chem 275:2130-6.
46. Rosa, R., C. Marques, O. R. Rodrigues, and G. M. Santos-Gomes. 2007. Immunization with Leishmania infantum released proteins confers partial protection against parasite infection with a predominant Th1 specific immune response. Vaccine 25:4525-32.
47. Santos-Gomes, G. M.-, R. Rosa, C. Leandro, S. Cortes, P. Romao, and H. Silveira. 2002. Cytokine expression during the outcome of canine experimental infection by Leishmania infantum. Vet Immunol Immunopathol 88:21-30.
48. Santos, W. R., V. M. de Lima, E. P. de Souza, R. R. Bernardo, M. Palatnik, and C. B. Palatnik de Sousa. 2002. Saponins, IL12 and BCG adjuvant in the FML-vaccine formulation against murine visceral leishmaniasis. Vaccine 21:30-43.
49. Saraiva, E. M., A. de Figueiredo Barbosa, F. N. Santos, G. P. Borja-Cabrera, D. Nico, L. O. Souza, C. de Oliveira Mendes-Aguiar, E. P. de Souza, P. Fampa, L. E. Parra, I. Menz, J. G. Dias, Jr., S. M. de Oliveira, and C. B. Palatnik-de-Sousa. 2006. The FML-vaccine (Leishmune) against canine visceral leishmaniasis: a transmission blocking vaccine. Vaccine 24:2423-31.
50. Serezani, C. H., A. R. Franco, M. Wajc, J. K. Umada Yokoyama-Yasunaka, G. Wunderlich, M. M. Borges, and S. R. Uliana. 2002. Evaluation of the murine immune response to Leishmania meta 1 antigen delivered as recombinant protein or DNA vaccine. Vaccine 20:3755-63.
51. Skeiky, Y. A., J. A. Guderian, D. R. Benson, O. Bacelar, E. M. Carvalho, M. Kubin, R. Badaro, G. Trinchieri, and S. G. Reed. 1995. A recombinant Leishmania antigen that stimulates human peripheral blood mononuclear cells to express a Th1-type cytokine profile and to produce interleukin 12. J Exp Med 181:1527-37.
52. Stager, S., D. F. Smith, and P. M. Kaye. 2000. Immunization with a recombinant stage-regulated surface protein from Leishmania donovani induces protection against visceral leishmaniasis. J Immunol 165:7064-71.
54. Tonui, W. K., J. S. Mejia, L. Hochberg, M. L. Mbow, J. R. Ryan, A. S. Chan, S. K. Martin, and R. G. Titus. 2004. Immunization with Leishmania major exogenous antigens protects susceptible BALB/c mice against challenge infection with L. major. Infect Immun 72:5654-61.
55. Webb, J. R., A. Campos-Neto, Y. A. Skeiky, and S. G. Reed. 1997. Molecular characterization of the heat-inducible LmSTI1 protein of Leishmania major. Mol Biochem Parasitol 89:179-93.
56. Webb, J. R., D. Kaufmann, A. Campos-Neto, and S. G. Reed. 1996. Molecular cloning of a novel protein antigen of Leishmania major that elicits a potent immune response in experimental murine leishmaniasis. J Immunol 157:5034-41.
57. Zimmermann, S., O. Egeter, S. Hausmann, G. B. Lipford, M. Rocken, H. Wagner, and K. Heeg. 1998. CpG oligodeoxynucleotides trigger protective and curative Th1 responses in lethal murine leishmaniasis. J Immunol 160:3627-30.

## Claims

1. Use of a Ribosomal Protein Extract and optionally a Th₁-promoting adjuvant for the preparation of a medicament for the treatment or prevention of a parasitic disease in a subject.

2. Use according to claim 1, wherein the medicament is a vaccine.

3. Use accoding to claim 1 or 2, wherein the Ribosomal Protein Extract is obtainable by carrying out the following steps using a parasite cell causing a parasitic disease when present in a subject:
a. mixing a parasite cell with a lysis buffer,
b. centrifuging the obtained mixture to obtain a cytosolic extract,
c. preparing the Ribosomal Protein Extract from the obtained cytosolic extract.

4. Use according to any one of claims 1 to 3, wherein the Ribosomal Protein Extract is obtained from a *Leishmania* species, preferably *Leishmania major.*

5. Use according to any one of claims 1 to 4, wherein the Th₁-promoting adjuvant is a CpG ODN.

6. Use according to any one of claims 1 to 5, wherein the parasitic disease is leishmaniasis or malaria.

7. Use acording to any one of claims 1 to 6, wherein the parasitic disease is caused by a *Leishmania* or by a *Plasmodium* species.

8. Use according to any one of claims 1 to 7, wherein the parasitic disease is caused by a different species than the species from which the Ribosomal Protein Extract is derived.

9. A composition comprising a Ribosomal Protein Extract and optionally a Th₁-promoting adjuvant.

10. A composition according to claim 9, consisting of a Ribosomal Protein Extract and a Th₁-promoting adjuvant.

11. A composition according to claim 9 or 10, wherein the Th₁-promoting adjuvant is a CpG ODN.

12. A composition according to any one of claim 9 to 11, further comprising a pharmaceutically acceptable adjuvant and/or carrier.

13. A composition according to any one of claim 9 to 12, for use as a medicament.

14. A composition according to claim 12, wherein the medicament is a vaccine.
